# EUROPÄISCHE PATENTSCHRIFT

(11) **EP 1 314 405 B1**
(45) Veröffentlichungstag und Bekanntmachung des Hinweises auf die Patenterteilung: **11.04.2007**
(21) Anmeldenummer: 02090344.9
(22) Anmeldetag: 26.09.2002
(51) Int. Cl.: A61F 2/06

(54) **Implantat mit proliferationshemmender Substanz**
Graft with proliferation-inhibiting substance
Implant avec substance pour inhiber la prolifération

(30) Priorität: 08.10.2001 DE 10150995
(43) Veröffentlichungstag der Anmeldung: 28.05.2003
(73) Patentinhaber: BIOTRONIK GmbH & Co. KG, 12359 Berlin (DE)
(72) Erfinder: Stoll, Hans-Peter, 52146 Würselen (DE)
(74) Vertreter: Lindner-Vogt, Karin L.

(56) Entgegenhaltungen:
- EP-A- 0 879 595
- US-A- 5 980 551
- US-A- 6 096 070

## Beschreibung

Die vorliegende Erfindung betrifft ein Implantat zum Einsetzen in einen Abschnitt des menschlichen oder tierischen Blutkreislaufs, insbesondere einen Stent, mit einem Grundkörper, wenigstens einem mit dem Grundkörper verbundenen, wenigstens eine zellproliferationshemmende erste Substanz umfassenden ersten Stoff und einem sich im implantierten Zustand zersetzenden zweiten Stoff. Der zweite Stoff ist dabei derart angeordnet und ausgebildet, dass er in einem ersten Zustand vor Beginn seiner Zersetzung den Kontakt zwischen der ersten Substanz und der Umgebung des Grundkörpers verhindert und in einem zweiten Zustand nach Beginn seiner Zersetzung den Kontakt zwischen der ersten Substanz und der Umgebung des Grundkörpers ermöglicht.

Bei solchen intravaskulären oder intrakardialen Implantaten, wie Stents, Elektrodenleitungen oder dergleichen besteht häufig das Problem, dass durch den nicht ausreichend biokompatiblen Fremdkörper, den das Implantat darstellt, oder durch beim Implantieren entstehende Verletzungen des angrenzenden Körpergewebes ein unkontrolliertes Zellwachstum im umliegenden Körpergewebe des Patienten induziert wird.

Dieses unkontrollierte Zellwachstum ist unerwünscht. So kann es beispielsweise bei intravaskulären Implantaten dazu führen, dass die Gefäßbahn durch das wuchernde Gewebe stark verengt oder gar über kurz oder lang vollständig verschlossen wird. Dies ist in jedem Fall zu verhindern, da eine solche Gefäßverengung bzw. ein solcher Gefäßverschluss unter Umständen einen lebensbedrohlichen Zustand des Patienten herbeiführen kann.

Um diesem Problem zu begegnen ist beispielsweise aus der US 6,153,252, der US 6,206,916 B1, und der US 5,980,551 jeweils ein gattungsgemäßes Implantat in Form eines Stents bekannt, bei dem durch eine bioresorbierbare Beschichtung des Stents eine zellproliferationshemmende Substanz abgegeben wird, um das unkontrollierte Zellwachstum der Gefäßwand im Bereich des Stents zu unterdrücken. Um die Wirkstoffabgabe zu verzögern oder langsamer vonstatten gehen zu lassen ist dabei unter anderem vorgesehen, eine bioresorbierbare Deckschicht über der wirkstofftragenden Beschichtung anzubringen.

Die bei den bekannten Stents verwendeten zellproliferationshemmenden Substanzen sind in der Regel derart wirksam, dass es zu keinen nennenswerten Zellwachstum in der Umgebung des Implantats kommt.

Ein weiteres Problem im Zusammenhang mit solchen intravaskulären oder intrakardialen Implantaten besteht in ihrer thrombogenen Wirkung, also dem mit ihnen einhergehenden erhöhten Thromboserisiko. Je nach Grad ihrer Biokompatibilität kann es an ihrer Oberfläche vergleichsweise leicht zur Bildung von Thromben kommen. Diese können zum einen mit den oben bereits genannten Konsequenzen ebenfalls zu einer Verengung bzw. zu einem Verschluss des betreffenden Gefäßabschnittes führen. Zum anderen können sich derartige Thromben auch von ihrem Entstehungsort lösen und mit dem Blutstrom zu entfernten kleineren Blutgefäßen transportiert werden, wo sie dann zu einer so genannten Embolie, d. h. einer Verengung bzw. Verstopfung führen können.

Um dem Problem der Thrombenbildung vorzubeugen, ist bei bekannten Implantaten, insbesondere bei den oben genannten Stents vorgesehen, in der oben geschilderten Weise auch eine oder mehrere antithrombogene Substanzen aus einer entsprechenden Beschichtung des Stents freizusetzen.

Hierbei besteht jedoch das Problem, dass die antithrombogene Substanz in der Regel auf Grund der ständig vorhandenen Blutströmung zum einen nach ihrer Freisetzung vergleichsweise schnell abtransportiert wird und daher nur eine geringe Wirkung erzielen kann. Dieser zur Folge, dass eine relativ hohe Menge der antithrombogenen Substanz in der Beschichtung gebunden sein und freigesetzt werden muss, um eine der Bildung von Thromben verhindernde Wirkung zu erzielen.

Zum anderen bewirkt die ständige Blutströmung, dass die wirkstofftragende Beschichtung auch relativ schnell ausgewaschen bzw. abgetragen wird, sodass die erforderliche langanhaltende Medikamentierung mit antithrombogener Wirkung nicht oder nur mit erheblichem Aufwand beispielsweise durch eine Mehrfachbeschichtung mit unterschiedlichen Wirkstoffkonzentrationen oder Abgaberaten erzielt werden kann.

Der vorliegenden Erfindung liegt daher die Aufgabe zu Grunde, ein gattungsgemäßes Implantat anzugeben, welches mit einfachen Mitteln eine langanhaltende Verringerung der Gefahr der Thrombenbildung ermöglicht.

Die vorliegende Erfindung löst diese Aufgabe ausgehend von einem Implantat gemäß dem Oberbegriff des Anspruchs 1 durch die im kennzeichnenden Teil des Anspruchs 1 angegebenen Merkmale.

Der vorliegenden Erfindung liegt die technische Lehre zu Grunde, dass man eine einfache und langanhaltende Reduktion des von einem solchen Implantat ausgehenden Thromboserisikos erzielt, wenn man zunächst die Zellproliferation in der Umgebung des Implantats solange zulässt, bis sich eine das Implantat zumindest teilweise einhüllende Körpergewebelage gebildet hat. Diese Körpergewebelage bildet dann auf Grund ihres Aufbaus aus körpereigenen Zellen eine dauerhafte, das Risiko der Thrombenbildung erheblich reduzierende Schicht bzw. Hülle über dem Implantat. Erst nach der Bildung dieser Körpergewebelage wird eine zellproliferationshemmende Substanz zur Wirkung gebracht, um ein über die Bildung der Körpergewebelage hinausgehendes unkontrolliertes Zellwachstum zu verhindern.

Erfindungsgemäß wird dies dadurch erreicht, dass der zweite Stoff derart ausgebildet ist, dass der zweite Zustand, in dem die zellproliferationshemmende erste Substanz in Folge der Zersetzung des zweiten Stoffes zur Wirkung kommen kann, erst nach einem ersten Zeitintervall erreicht wird, welches zur Bildung einer wenigstens einen Abschnitt der Oberfläche des Grundkörpers einhüllenden Körpergewebelage ausreicht.

Die Körpergewebelage umhüllt dabei bevorzugt zumindest in dem Bereich die restliche Oberfläche des Implantats, in dem das Implantat im frisch implantierten Zustand, d. h. unmittelbar nach der Implantation das umliegende Körpergewebe kontaktiert. Vor allem in diesem Bereich kann es zu entsprechendem Zellwachstum kommen, welches die Bildung der Körpergewebelage ermöglicht.

Im Fall eines Stents handelt es sich bei dem einzuhüllenden Abschnitt somit bevorzugt um die restliche Oberfläche des Stents in dem Bereich, in dem die äußere Oberfläche des Stents die Gefäßwand kontaktiert. Da ein Stent in der Regel über seine gesamte Länge an dem Gefäß anliegt, handelt es sich bei dem durch die Körpergewebelage einzuhüllenden Abschnitt regelmäßig um die gesamte restliche, die Gefäßwand nicht unmittelbar kontaktierende Oberfläche des Stents.

Die Länge des ersten Zeitintervalls bestimmt sich nach den am jeweiligen Implantationsort des jeweiligen Patienten wirksamen Zellproliferationsmechanismen, insbesondere nach der jeweiligen Geschwindigkeit des Zellwachstums. Diese können von Implantationsort zu Implantationsort sowie von Patient zu Patient verschieden sein, lassen sich jedoch in Versuchen ohne weiteres ermitteln. Je schneller das Zellwachstum an dem betreffenden Ort bei dem betreffenden Patienten vonstatten geht, desto kürzer ist das erste Zeitintervall.

Die Patienten können gegebenenfalls nach unterschiedlichen, das Zellwachstum beeinflussenden Faktoren, wie beispielsweise Alter, sonstiger Disposition aber auch anderweitiger Behandlung bzw. Medikamentierung, in unterschiedliche Kategorien eingeteilt werden, für die unterschiedliche Längen des ersten Intervalls vorgesehen sind. Gegebenenfalls ist es sogar möglich, anhand entsprechender Untersuchungen das erste Intervall auf den einzelnen Patienten abzustimmen, d. h. für den jeweiligen Patienten ein maßgeschneidertes Implantat zur Verfügung zu stellen.

Das erste Zeitintervall reicht vorzugsweise zur Bildung einer einen Abschnitt der Oberfläche des Grundkörpers bedeckenden Hülle aus Körpergewebe aus. Hierbei handelt es sich, insbesondere im Falle eines in ein Blutgefäß implantierten Stents, vorzugsweise um eine Hülle aus glatten Muskelzellen. Schon eine relativ dünne derartige Körpergewebeschicht ist für die Erfüllung des zu erzielenden Zweckes einer Abdeckung der thrombogenen Oberfläche des Grundkörpers völlig ausreichend.

Je nach Implantat ist bevorzugt vorgesehen, das erste Intervall solange zu wählen, dass die erzielte Körpergewebelage möglichst die gesamte blutumströmte Oberfläche des Grundkörpers bedeckt, um das Risiko der Thrombenbildung an offenliegenden Oberflächenbereichen des Grundkörpers möglichst weitgehend zu reduzieren.

Die Länge des ersten Zeitintervalls hängt, wie oben erwähnt, von unterschiedlichen Faktoren ab. Um sicherzustellen, dass sich eine möglichst zusammenhängende, die Oberfläche des Grundkörpers möglichst umfassend abdeckende Körpergewebelage erzielt wird, beträgt das erste Zeitintervall bevorzugt wenigstens eine Woche. Um weiterhin sicherzustellen, dass es zu keiner allzu dicken Körpergewebelage kommt ist weiter vorzugsweise vorgesehen, dass das erste Zeitintervall zwei bis vier Wochen beträgt.

Der erste Stoff bzw. wenigstens eine seiner Komponenten kann sowohl biostabil als auch bioresorbierbar sein, d. h. sich im implantierten Zustand auf Grund der Einwirkung von körpereigenen Stoffen zersetzen.

Der erste Stoff kann aus einem Material bestehen, welches für sich eine entsprechend zellproliferationshemmende Wirkung aufweist. Mit anderen Worten kann der erste Stoff ausschließlich aus der ersten Substanz bestehen.

Der erste Stoff ist bevorzugt jedoch nach Art eines Verbundwerkstoffes aufgebaut, da sich ein solcher Verbund besonders gut an die vielfältigen Anforderungen an die chemischen und physikalischen, insbesondere die mechanischen Eigenschaften des ersten Stoffes anpassen lässt.

Der erste Stoff umfasst hierzu vorteilhafterweise eine erste Komponente, welche eine Matrix bildet, in der wiederum die zellproliferationshemmende erste Substanz eingelagert ist. Die Einlagerung kann dabei in einem einfachen Fall letztlich durch rein mechanische Einbindung der ersten Substanz in der Matrix aus der ersten Komponente erfolgen. Es kann sich bei anderen Varianten aber ebenso auch um eine chemische Bindung sowie eine Kombination aus chemischer und mechanischer Bindung zwischen der ersten Substanz und der ersten Komponente handeln.

Die erste Substanz kann so gewählt bzw. ausgebildet sein, dass sie ihre zellproliferationshemmende Wirkung allein durch entsprechenden Kontakt entfaltet, ohne dass sie hierzu aus ihrem Verbund innerhalb des ersten Stoffes herausgelöst werden muss.

Besonders gute Ergebnisse lassen sich jedoch erzielen, wenn die erste Substanz in entsprechend kleinen Mengen dosiert an die Umgebung abgegeben wird. Dies lässt sich beispielsweise dadurch erzielen, dass die erste Substanz selbst bioresorbierbar ist oder der Verbund zwischen Matrix und erster Substanz, beispielsweise durch langsame Zersetzung der Matrix, aufgelöst wird.

Die Freisetzungsrate sowie deren zeitlicher Verlauf, also das Freisetzungsprofil, der ersten Substanz können in bekannter Weise in weiten Grenzen beeinflusst werden. So kann beispielsweise vorgesehen sein, dass durch einen Konzentrationsgradienten der ersten Substanz innerhalb des ersten Stoffes oder durch eine Anordnung des ersten Stoffes in mehreren Schichten mit unterschiedlichen Konzentrationen der ersten Substanz zu bestimmten Zeiten unterschiedliche Mengen der ersten Substanz freigesetzt werden.

Beispielsweise kann durch die Zersetzung einer äußeren Schicht mit relativ hoher Konzentration der ersten Substanz zunächst eine vergleichsweise große Menge der ersten Substanz pro Zeiteinheit freigesetzt werden, um die Zellproliferation möglichst schnell zu hemmen. Nach einer bestimmten Zeit, die zum im wesentlichen vollständigen Abbau dieser äußeren Schicht benötigt wird, mit anderen Worten also durch die Dicke der Schicht bedingt ist, beginnt dann der Abbau einer darunter liegenden Schicht mit einer geringeren Konzentration der ersten Substanz. Hierbei wird dann nur noch eine geringere Menge der ersten Substanz pro Zeiteinheit freigegeben. Dies kann beispielsweise sinnvoll sein, wenn sich der mit der Anwesenheit des Implantats verbundene Zellproliferationsstimulus nach einer gewissen Zeit in Folge einer "Gewöhnung" an den Fremdkörper abschwächt und verhindert werden soll, dass die über die Zeit notwendige Regeneration der erwünschten Körpergewebelage durch eine allzu hohe Dosis ebenfalls blockiert wird.

Eine weitere Möglichkeit, die Freisetzungsrate zu beeinflussen liegt in der Wahl der Zusammensetzung der die Matrix bildenden ersten Komponente. So kann vorgesehen sein, an Stelle der Konzentration der ersten Substanz die Beschaffenheit der Matrix innerhalb des ersten Stoffes zu variieren.

Um hierbei wiederum das Freisetzungsprofil auf den jeweiligen Bedarf einzustellen, können in einem einfachen Fall mehrere Schichten vorgesehen sein, deren Matrix sich unterschiedlich schnell zersetzt, um zu bestimmten Zeitpunkten unterschiedliche Freisetzungsraten zu erzielen. Es versteht sich jedoch, dass die unterschiedliche Beschaffenheit der Matrix auch durch Variationen innerhalb einer zusammenhängenden Schicht erzielt werden kann, beispielsweise durch eine Variation der Dichte oder des Molekulargewichtes etc. der Matrix.

Mit den soeben beschriebenen Varianten lassen sich beliebige Freisetzungsprofile erzielen, die auf die spezielle Disposition und Behandlung des Patienten, den Implantationsort sowie den gewünschten Behandlungserfolg abgestimmt sein können. Es versteht sich, dass die Variation der Konzentration der ersten Substanz sowie die Variation der Beschaffenheit der Matrix natürlich auch in beliebiger Weise miteinander kombiniert werden können.

Es versteht sich, dass bei vorteilhaften Varianten des erfindungsgemäßen Implantats auch vorgesehen sein kann, dass der erste Stoff mehrere unterschiedliche zellproliferationshemmende erste Substanzen enthält, um eine auf die spezielle Disposition und Behandlung des Patienten, den Implantationsort sowie den gewünschten Behandlungserfolg abgestimmte zellproliferationshemmende Wirkung zu erzielen.

Eine weitere Möglichkeit der Einflussnahme auf den zeitlichen Verlauf der zellproliferationshemmenden Wirkung des Implantats besteht demgemäß auch darin, unterschiedliche erste Substanzen zu verwenden, die unterschiedlich stark zellproliferationshemmend wirken. Diese können dann wieder ähnlich den oben genannten Varianten in unterschiedlichen aufeinander folgenden Schichten eingebunden sein, um zu unterschiedlichen Zeiten eine unterschiedlich starke Hemmung der Zellproliferation zu erzielen.

Der erste Stoff bzw. wenigstens eine seiner Komponenten kann sowohl biostabil als auch bioresorbierbar sein, d. h. sich im implantierten Zustand auf Grund der Einwirkung von körpereigenen Stoffen zersetzen.

Der erste Stoff umfasst wie erwähnt bevorzugterweise wenigstens eine erste Komponente, welche eine Matrix bildet, in der wiederum die erste Substanz eingelagert ist. Es versteht sich jedoch, dass für die Matrix auch mehrere unterschiedliche Komponenten vorgesehen sein können. Für die Matrix finden bevorzugt entsprechend biokompatible Polymere ihre Anwendung, da deren Eigenschaften besonders einfach auf die obengenannten Anforderungen eingestellt werden können.

Bei dem zweiten Stoff handelt es sich um ein bioresorbierbares Material, das aus einer oder mehreren Komponenten bestehen kann, die mit keiner der Komponenten des ersten Stoffes stoffgleich ist bzw. sind. Der zweite Stoff kann aber ebenso gut mit einer Komponente des ersten Stoffes stoffgleich sein. Bei einem Aufbau des ersten Stoffes aus einer Matrix, in der die erste Substanz eingelagert ist, kann der zweite Stoff beispielsweise aus der oder einer der die Matrix bildenden Komponenten des ersten Stoffes bestehen bzw. dieser entsprechen.

Beispiele geeigneter Materialien für den ersten Stoff bzw. seine Komponenten, insbesondere für die zellproliferationshemmende erste Substanz, ebenso wie für den zweiten Stoff sind hinlänglich bekannt, sodass an dieser Stelle hierauf nicht näher eingegangen werden soll. Eine Reihe geeigneter Materialien ergibt sich beispielsweise aus den beiden eingangs genannten Dokumenten US 6,153,252 und US 6,206,916 B1.

Bei vorteilhaften Varianten des erfindungsgemäßen Implantats ist wenigstens ein erstes Depot des ersten Stoffes vorgesehen, welches im ersten Zustand durch eine Abdeckung des zweiten Stoffes gegenüber der Umgebung des Grundkörpers abgeschirmt ist. Die Abdeckung muss den ersten Stoff dabei nicht notwendigerweise gegen den Grundkörper abschirmen, sie muss lediglich sicherstellen, dass es zu keinem, zumindest keinem nennenswerten Kontakt mit der Umgebung des Grundkörpers kommt, um zu verhindern, dass eine nennenswerte zellproliferationshemmende Wirkung vor Ablauf des ersten Zeitintervalls eintritt.

Das erste Depot kann in beliebiger Weise mit dem Grundkörper verbunden sein. Hierbei muss keine unmittelbare Verbindung zwischen dem ersten Depot und den Grundkörper bestehen, sondern die Verbindung kann auch mittelbar über weitere Zwischenelemente bestehen.

Das erste Depot kann in vielfältiger Weise gestaltet sein. Um eine über das Implantat oder zumindest über dessen im vorliegenden Zusammenhang relevante Bereiche gleichmäßig verteilte zellproliferationshemmende Wirkung zu erzielen, weist das erste Depot entweder eine entsprechende Ausdehnung auf oder es ist eine Reihe entsprechend über das Implantat verteilter erster Depots vorgesehen.

Bei einfachen Varianten des erfindungsgemäßen Implantats ist vorgesehen, dass das erste Depot von einer mit dem Grundkörper verbundenen ersten Schicht gebildet ist, während die Abdeckung von einer die erste Schicht bedeckenden zweiten Schicht gebildet ist. Die erste Schicht kann dabei den gesamten Grundkörper bedecken, sodass sich das erste Depot über das gesamte Implantat erstreckt. Sie kann aber auch nur einen bestimmten Bereich der Oberfläche des Grundkörpers bedecken. Es ist jeweils lediglich sicherzustellen, dass die zweite Schicht die erste Schicht, wie oben dargelegt, ausreichend gut gegenüber der Umgebung abschirmt.

Das erste Depot ist bei weiteren bevorzugten Varianten des erfindungsgemäßen Implantats in einer ersten Ausnehmung des Grundkörpers angeordnet. Die Abdeckung ist dann von einem zumindest die erste Ausnehmung verschließenden bzw. abdeckenden Deckel gebildet. Hierbei kann das Depot teilweise über die Oberfläche des Grundkörpers hinausragen, bevorzugt ist jedoch vorgesehen, dass das erste Depot im Grundkörper versenkt angeordnet ist. Vergleichbares gilt für den Deckel. Dieser kann beispielsweise so angeordnet sein, dass seine äußere Oberfläche zumindest im Randbereich der ersten Ausnehmung bündig mit der Oberfläche des Grundkörpers abschließt. Ebenso kann es allerdings vorgesehen sein, dass der Deckel von einer zusammenhängenden Beschichtung des Grundkörpers gebildet ist, die mehrere erste Depots verschließt bzw. abdeckt. Schließlich kann natürlich auch vorgesehen sein, dass der jeweilige Deckel von einer punktuellen Beschichtung des Grundkörpers gebildet ist.

Durch die Anordnung des ersten Depots in der ersten Ausnehmung kann zum einen eine relativ einfache Verankerung des ersten Stoffes erzielt werden. Zum anderen lassen sich insofern besonders vorteilhafte Anordnungen erzielen, als das Depot je nachdem, ob es ganz oder teilweise in dem Grundkörper versenkt ist, ganz oder teilweise vor Querkräften geschützt ist, die andernfalls in beim Heranführen des Implantats an die Implantationsstelle entgegen der Bewegungsrichtung des Implantats auf das Depot wirken könnten.

Sind die ersten Depots in solchen ersten Ausnehmungen angeordnet, liegt ein weiterer Vorteil darin, dass das bei einer Verformung des Grundkörpers, wie sie regelmäßig beispielsweise bei Stents auftritt, ein geringeres Risiko der Beschädigung ihrer Abdeckung durch den zweiten Stoff besteht. Dies rührt daher, dass es auf Grund der diskreten Anordnung der ersten Depots gegenüber Varianten mit ersten Depots aus einer großflächigeren zusammenhängenden Schicht auf die Oberfläche des Grundkörpers bezogen ein geringerer Flächenanteil mit Paarungen aus dem ersten und zweiten Stoff bedeckt ist. Insgesamt kann es also nur im Bereich eines geringeren Anteils der Oberfläche des Grundkörpers zu einer Beschädigung, beispielsweise einem Aufreißen, der Abdeckung des ersten Stoffes kommen.

Weitere Vorteile in dieser Richtung ergeben sich, wenn die jeweilige Ausnehmung von einem gesonderten Deckel verschlossen ist, da bei Verformung des Grundkörpers dann im Bereich der Paarung aus Deckel und Depot vergleichsweise geringe Verformungen auftreten, die zu einer vergleichsweise geringen Belastung des Deckels bzw. der Paarung aus Deckel und Depot führen, wodurch sich das Risiko einer Beschädigung der Abdeckung weiter reduziert.

Die erste Ausnehmung kann in beliebiger Weise in den Grundkörper eingebracht sein. Sie kann schon unmittelbar bei der Herstellung des Grundkörpers eingebracht werden. Sie kann aber auch durch einen entsprechenden Bearbeitungsschritt eines zuvor hergestellten Grundkörpers erzeugt werden. Dies kann beispielsweise durch eine spanende oder spanlose Bearbeitung des Grundkörpers geschehen. Hierbei können beispielsweise entsprechende Ätzverfahren oder eine Bearbeitung mittels Laser oder dergleichen ihren Einsatz finden. Ebenso ist es allerdings möglich, die erste Ausnehmung durch eine entsprechend beschaffene bzw. entsprechend verteilt angeordnete Beschichtung eines Grundkörperrohlings zu erzielen.

Die ersten Ausnehmungen können durch gezielte Bearbeitung des Grundkörpers gleichmäßig über das Implantat verteilt werden, um über den gesamten relevanten Bereich des Implantats eine möglichst gleichmäßige zellproliferationshemmende Wirkung zu erzielen. Besonders einfach lassen sich gleichmäßig über den Grundkörper verteilte erste Depots allerdings dann erzielen, wenn der Grundkörper zur Ausbildung einer Anzahl erster Ausnehmungen zumindest abschnittsweise eine poröse Oberfläche aufweist.

Bei anderen vorteilhaften Ausführungen des erfindungsgemäßen Implantats ist vorgesehen, dass das erste Depot in einer die Abdeckung bildenden Mikrokapsel aus dem zweiten Stoff eingeschlossen ist, welche mit dem Grundkörper verbunden ist. Diese Variante bietet den Vorteil, dass das Risiko einer Beschädigung der Abdeckung des ersten Stoffes durch den zweiten Stoff bei einer Verformung des Grundkörpers stark reduziert ist. Die Mikrokapsel kann der Verformung des Grundkörpers ohne weiteres folgen, ohne dass es zu einer starken Beanspruchung ihrer Wandung kommt.

In der Regel ist eine relativ große Anzahl solcher Mikrokapseln über das Implantat bzw. den hier maßgeblichen Bereich des Implantats verteilt. Diese Mikrokapseln enthalten dann jeweils nur eine vergleichsweise geringe Menge des ersten Stoffes. Selbst wenn bei einer Verformung des Implantats dann einzelne Mikrokapseln beschädigt werden sollten, kommt es zu keiner nennenswerten zellproliferationshemmenden Wirkung, da bestenfalls nur die in der betreffenden Mikrokapsel enthaltene, vergleichsweise geringe Menge der ersten Substanz ihrer Wirkung entfalten kann.

Die Mikrokapseln können in einer oder mehreren Schichten auf der Oberfläche des Grundkörpers angeordnet sein. Bei einer entsprechend dichten Packung der Mikrokapseln in mehreren Schichten kann eine langsame Freisetzung der ersten Substanz über einen bestimmten Zeitraum erzielt werden, da sich zumindest bevorzugt zunächst die weiter außen liegenden Mikrokapseln zersetzen und nachfolgend die darin enthaltene erste Substanz freigegeben wird.

Bei anderen Varianten kann die dosierte Freisetzung der ersten Substanz dadurch erfolgen, dass die Mikrokapseln unterschiedlich große Wandstärken aufweisen, d. h. die Freisetzung der ersten Substanz je nach Wandstärke erst nach einer bestimmten Verzögerung beginnen kann. Derselbe Effekt lässt sich erzielen, wenn für die Mikrokapseln unterschiedliche Materialien verwendet werden, die sich unterschiedlich schnell zersetzen.

Die Mikrokapseln können ihrerseits auch in entsprechenden ersten Ausnehmungen des Grundkörpers angeordnet bzw. akkumuliert sein. Diese Anordnung vereint die Vorteile der Mikrokapseln mit den oben geschilderten Vorteilen der Anordnung des ersten Depots in derartigen ersten Ausnehmungen. Insbesondere sind die Mikrokapseln in diesen Ausnehmungen vor der Einwirkung äußerer Kräfte und damit ihrer Beschädigung bzw. Entfernung vom Implantat geschützt.

Die Anbindung der Mikrokapseln an den Grundkörper und gegebenenfalls ihre Verbindung untereinander kann in beliebiger bekannter Weise erfolgen. So können die Mikrokapseln beispielsweise durch einen entsprechenden Klebstoff oder dergleichen miteinander und/oder mit dem Grundkörper verbunden sein. Ebenso kann natürlich vorgesehen sein, dass die Mikrokapseln in einem sinterartigen Verfahren durch ein partielles Aufschmelzen ihrer Oberfläche miteinander und/oder mit den Grundkörper verbunden werden.

Es versteht sich, dass die soeben beschriebenen Varianten der Anordnung und Gestaltung des ersten Depots auch in beliebiger Weise miteinander kombiniert werden können. So können z. B. in mechanisch stark beanspruchten Bereichen Mikrokapseln vorgesehen sein, während in weniger stark beanspruchten Bereichen großflächigere bzw. großvolumigere Depots der weiter oben beschriebenen Arten vorgesehen sind.

Weitere bevorzugte Ausgestaltungen des erfindungsgemäßen Implantats zeichnen sich dadurch aus, dass zur Förderung der Zellproliferation vor Ablauf des ersten Zeitintervalls ein mit dem Grundkörper verbundener dritter Stoff vorgesehen ist, der eine zellproliferationsfördernde zweite Substanz umfasst. Hierdurch kann erreicht werden, dass die das Risiko einer Thrombenbildung reduzierende erste Körpergewebelage möglichst gezielt und schnell gebildet wird. Bei diesen Varianten kann das erste Zeitintervall natürlich auf Grund der erzielten Beschleunigung der Bildung der ersten Körpergewebelage entsprechend kürzer ausfallen.

Der dritte Stoff kann in wenigstens einem zweiten Depot angeordnet sein. Dieses zweite Depot kann in derselben Weise wie das oben beschriebene erste Depot angeordnet und ausgebildet sein. Hierbei kann auch vorgesehen sein, dass das zweite Depot mit einer entsprechenden Abschirmung durch einen weiteren, vierten Stoff versehen ist, die ein um ein zweites Zeitintervall verzögertes Einsetzen der zellproliferationsfördernden Wirkung bedingt. Hierbei versteht es sich, dass der vierte Stoff dem zweiten Stoff entsprechen kann. Weiterhin versteht es sich, dass das zweite Zeitintervall entsprechend kürzer ist als das erste Zeitintervall.

Bevorzugt ist wiederum eine Anzahl zweiter Depots vorgesehen, die vorzugsweise entsprechend gleichmäßig über den Grundkörper bzw. den maßgeblichen Bereich des Grundkörpers verteilt sind, um eine möglichst gleichmäßige zellproliferationsfördernde Wirkung und damit eine möglichst gleichmäßige erste Körpergewebeschicht zu erzielen.

Es kann im übrigen auch vorgesehen sein, dass kein gesonderter dritter Stoff, sondern der die Abschirmung für den ersten Stoff bildende zweite Stoff dem dritten Stoff entspricht, mithin also der zweite Stoff die zellproliferationsfördernde zweite Substanz umfasst. Sobald dieser die zellproliferationsfördernde zweite Substanz umfassende zweite Stoff dann abgebaut ist und damit der erste Stoff frei liegt, setzt anschließend die Wirkung der zellproliferationshemmenden ersten Substanz ein.

Die zellproliferationsfördernde und die zellproliferationshemmende Wirkung können einander überschneiden, d. h. die Wirkung der zweiten Substanz kann noch anhalten, wenn die Wirkung der ersten Substanz schon einsetzt. Bei bevorzugten Varianten des erfindungsgemäßen Implantats ist der dritte Stoff derart ausgebildet, insbesondere die Konzentration der zweiten Substanz derart gewählt, dass die zellproliferationsfördernde Wirkung vor oder mit Ablauf des ersten Zeitintervalls im wesentlichen abgeschlossen ist.

Es versteht sich, dass bei weiteren Varianten der Erfindung durch entsprechende Gestaltung und/oder Anordnung erster und zweiter Depots auch erreicht werden kann, dass einander über die Zeit Phasen ablösen, in denen eine zellproliferationshemmende bzw. eine zellproliferationsfördernde Wirkung durch Freisetzung entsprechender erster bzw. zweiter Substanzen erzielt wird.

Die Erfindung lässt sich auf beliebige intravaskuläre oder intrakardiale Implantatstypen, wie z. B. Elektroden etc., anwenden. Besonders vorteilhaft lässt sie sich jedoch umsetzen, wenn es sich bei dem Implantat um einen Stent, insbesondere einen Koronarstent handelt.

Es versteht sich, dass die beschriebenen ersten, zweiten und dritten Stoffe in beliebiger bekannter Weise durch entsprechende Beschichtungsverfahren oder dergleichen auf das Implantat aufgebracht werden können. Insbesondere ist es möglich, dass einer oder mehrere dieser Stoffe, beispielsweise durch den behandelnden Chirurgen, unmittelbar vor der Operation durch ein häufig auch als "dip coating" bezeichnetes Tauchverfahren auf das Implantat aufgebracht werden.

Weitere vorteilhafte Ausführungen der Erfindung ergeben sich aus den Unteransprüchen bzw. der nachfolgenden Beschreibung bevorzugter Ausführungsbeispiele, welche auf die beigefügten Zeichnungen Bezug nimmt. Es zeigen:
- Figur 1: einen schematischen Teilschnitt durch ein bevorzugtes Ausführungsbeispiel des erfindungsgemäßen Implantats;
- Figur 2A und 2B: weitere schematische Teilschnitte durch das in den Körper eingesetzte Implantat aus Figur 1 zu unterschiedlichen Zeitpunkten;
- Figur 3: einen schematischen Teilschnitt durch ein weiteres bevorzugtes Ausführungsbeispiel des erfindungsgemäßen Implantats;
- Figur 4: einen schematischen Teilschnitt durch ein anderes bevorzugtes Ausführungsbeispiel des erfindungsgemäßen Implantats;
- Figur 5: das Detail V aus Figur 4;
- Figur 6: einen schematischen Teilschnitt durch ein weiteres bevorzugtes Ausführungsbeispiel des erfindungsgemäßen Implantats;^
- Figur 7: einen schematischen Teilschnitt durch ein weiteres bevorzugtes Ausführungsbeispiel des erfindungsgemäßen Implantats;
- Figur 8: einen schematischen Teilschnitt durch ein weiteres bevorzugtes Ausführungsbeispiel des erfindungsgemäßen Implantats;
- Figur 9: einen schematischen Teilschnitt durch ein weiteres bevorzugtes Ausführungsbeispiel des erfindungsgemäßen Implantats.

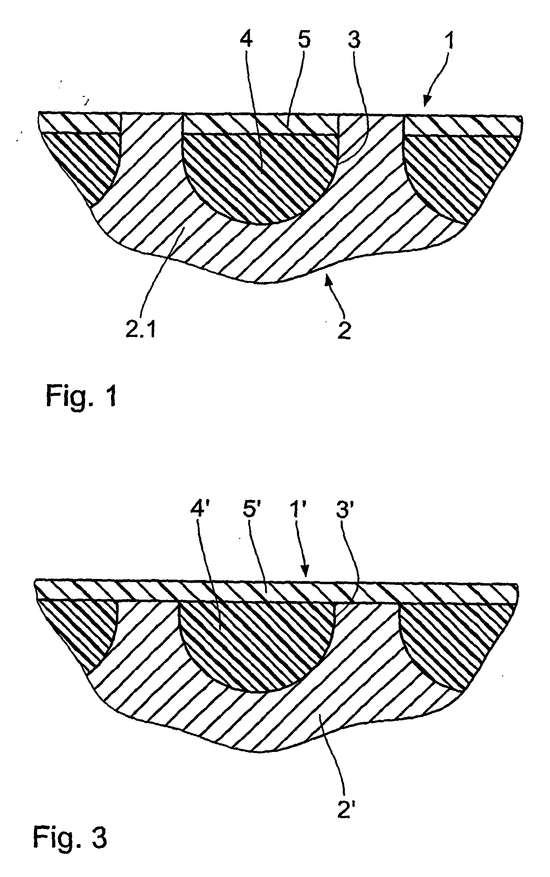

Figur 1 zeigt einen schematischen Teilschnitt durch ein bevorzugtes Ausführungsbeispiel eines erfindungsgemäßen Implantats in Form eines Stents 1, der einen Grundkörper 2 aufweist. Der Grundkörper 2 besitzt eine rohrförmige Gestalt, wobei der Mantel netzartig durchbrochen ist und aus Stegelementen 2.1 besteht, welche die Öffnungen im Mantel des Grundkörpers 2 umgrenzen.

Das in Figur 1 dargestellte Stegelement 2.1 weist eine Anzahl von ersten Ausnehmungen 3 auf. In diesen ersten Ausnehmungen 3 ist jeweils ein erstes Depot 4 aus einem ersten Stoff angeordnet. Das erste Depot 4 ist in einem ersten Zustand vor oder unmittelbar nach dem Implantieren gegenüber der Umgebung des Grundkörpers 2 durch eine Abdeckung in Form eines Deckels 5 aus einem zweiten Stoff abgeschirmt.

Die Depots 4 sind gleichmäßig über den gesamten Grundkörper 2 des Stents 1 verteilt. Dabei finden sie sich sowohl auf dem Abschnitt der Oberfläche des Grundkörpers 2, welcher der Wandung eines Blutgefäßes zugewandt ist, als auch auf dem Abschnitt der Oberfläche des Grundkörpers 2, welcher dem Lumen des betreffenden Blutgefäßes zugewandt ist.

Es versteht sich jedoch, dass bei anderen Varianten zum einen vorgesehen sein kann, dass die ersten Depots mehr oder weniger gleichmäßig über die gesamten Oberfläche des Stents verteilt sind. Ebenso ist es möglich, dass die Oberflächenabschnitte mit entsprechenden ersten Depots versehen sind, welche die Öffnungen im Mantel des Grundkörpers unmittelbar begrenzen. Schließlich ist es natürlich auch möglich, dass die genannten Oberflächenabschnitte allein oder in beliebigen Kombinationen mit entsprechenden ersten Depots versehen sein können.

Die ersten Ausnehmungen 3 sind durch eine entsprechende Bearbeitung, beispielsweise mittels eines Lasers, gezielt in den Grundkörper 2 eingebracht. Sie weisen eine definierte Geometrie auf. Es versteht sich jedoch, dass die ersten Ausnehmungen bei anderen Varianten des erfindungsgemäßen Implantats beispielsweise auch durch einen entsprechend porösen Oberflächenbereich des Grundkörpers gebildet sein können. Die ersten Depots können dann in den Poren des Grundkörpers angeordnet sein.

Das erste Depot 4 und der Deckel 5 sind derart in das Stegelement 2.1 des Grundkörpers 2 versenkt, dass die äußere Oberfläche des Deckels 5 bündig mit der Oberfläche des Grundkörpers 2 abschließt. Hierdurch ist sichergestellt, dass beim Heranführen des Stents 1 an die Implantationsstelle keine Querkräfte auf das erste Depot 4 oder den Deckel 5 wirken, welche deren jeweilige Verankerung am Grundkörper 2 beeinträchtigen könnten.

Ein weiterer Vorteil der Gestaltung mit einem gesonderten Deckel 5 für jedes erste Depot 4 liegt darin, dass das Risiko einer Beschädigung der Abdeckung des ersten Depots 4 durch den Deckel 5 reduziert ist, welches in Folge der Verformung des Stents beim Heranführen an die Implantationsstelle und vor allem beim Expandieren des Stents besteht. Dies rührt daher, dass die Stegelemente 2.1 im Bereich eines jeden einzelnen ersten Depots 4 nur relativ kleinen Verformungen unterworfen sind, wodurch sich auch lokal nur relativ geringe Belastungen des Deckels 5 bzw. des ersten Depots 4 ergeben.

Der erste Stoff besteht aus einer Matrix aus einem bioresorbierbaren Polymer, d. h. aus einem Polymer, welches unter Einwirkung körpereigener Stoffe des Patienten zersetzt wird. In der Matrix des ersten Stoffes sind Partikel einer ersten Substanz eingelagert, die zellproliferationshemmend wirkt. Der zweite Stoff besteht aus einem ebenfalls bioresorbierbaren Polymer, welches jedoch keine zellproliferationshemmende Wirkung, insbesondere keine entsprechenden Einlagerungen aufweist.

Die Gestalt, insbesondere die Dicke des Deckels 5 ist in Abhängigkeit von den Eigenschaften des zweiten Stoffes so gewählt, dass infolge der Zersetzung des Deckels 5 in einem zweiten Zustand der Kontakt zwischen der ersten Substanz und dem an den Stent 1 angrenzenden körpereigenen Gewebe bzw. den Stent 1 an dieser Stelle umspülenden Körperflüssigkeiten erst nach Ablauf eines ersten Zeitintervalls nach Implantieren des Stents 1 erfolgt.

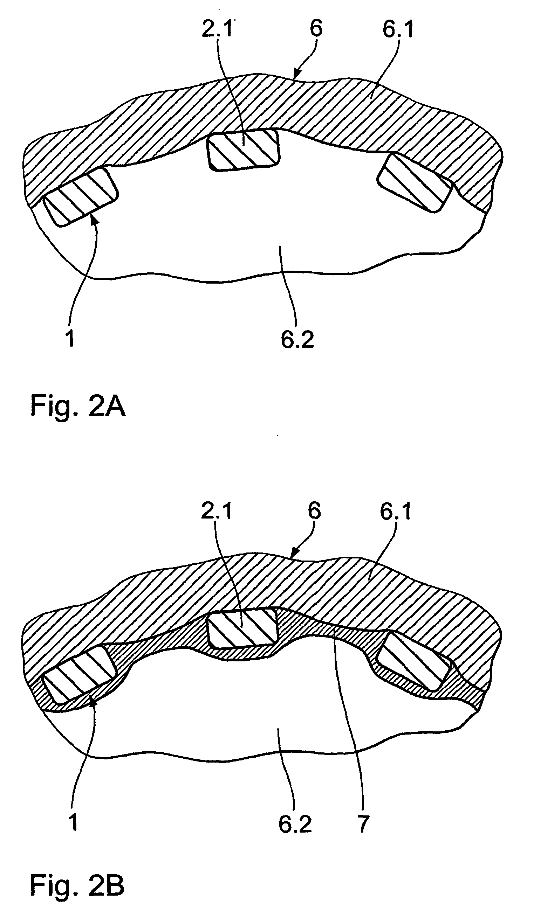

Die Figuren 2A und 2B zeigen den Stent 1 im ersten und zweiten Zustand. Die Figur 2A stellt dabei den ersten Zustand zum Zeitpunkt unmittelbar nach Expandieren des Stents 1 am Implantationsort im Blutgefäß 6 dar. Die Stegelemente 2.1 des Grundkörpers 2 kontaktieren über dessen gesamte Länge die Wandung 6.1 des Blutgefäßes 6 und halten dieses aufgeweitet, um einen normalen Blutstrom durch das Lumen 6.2 des Blutgefäßes 6 zu ermöglichen.

Das erste Zeitintervall ist so gewählt, dass sich nach seinem Ablauf, also bei Eintreten des zweiten Zustands, eine Körpergewebelage 7 gebildet hat, welche die Abschnitte der Oberfläche der Stegelemente 2.1 einhüllt, die nicht die Wandung 6.1 des Blutgefäßes 6 kontaktieren, wie dies in Figur 2B dargestellt ist . Diese Hülle 7 besteht im wesentlichen aus glatten Muskelzellen, welche hauptsächlich in Folge von Zellproliferationsmechanismen der tunica intima und media des Blutgefäßes 6 entstehen. Die Zellproliferationsmechanismen werden dabei hauptsächlich durch die Anwesenheit des Fremdkörpers induziert, den der Stent 1 darstellt.

Im vorliegenden Fall beträgt das erste Zeitintervall etwa 2 Wochen. Es versteht sich jedoch, dass je nach Disposition des Patienten, dessen Medikation und Implantationsort auch andere erste Zeitintervalle gewählt sein können.

Sobald durch Zersetzung der Polymermatrix des ersten Stoffes der ersten Depots 4 die zellproliferationshemmende erste Substanz freigesetzt wird, wird hierdurch das weitere Wachstum der Hülle 7 in einem Maße unterdrückt, welches sicherstellt, dass es zu keiner teilweisen oder gar vollständigen Obstruktion des Lumens 6.2 durch unkontrolliertes Zellwachstum kommt.

Im vorliegenden Fall ist vorgesehen, dass über das gesamte Depot 4 dieselbe Konzentration der ersten Substanz vorherrscht, die erste Substanz mit anderen Worten also gleichmäßig über das gesamte Depot 4 verteilt ist. Über die Zeit ergibt sich damit zunächst in etwa eine konstante Freisetzungsrate für die erste Substanz. Durch die bogenförmige Schnittkontur im Grund der Ausnehmungen 3 ergibt es sich jedoch, dass die bei der Zersetzung der Polymermatrix wirksame Oberfläche des Depots zu einem fortgeschrittenen Zeitpunkt immer kleiner wird, sodass die Freisetzungsrate der ersten Substanz ab einem bestimmten Zeitpunkt kontinuierlich abnimmt.

Die Polymermatrix des ersten Stoffes zerfällt nur sehr langsam, sodass die zellproliferationshemmende Wirkung über einen ausreichend langen Zeitraum anhält. Das langsame Ausklingen der zellproliferationshemmenden Wirkung kann dabei in Kauf genommen werden, da sich der Körper mit der Zeit an den Fremdkörper in Form des Stents 1 gewöhnt.

Die Hülle 7 bildet auf Grund ihres Aufbaus aus körpereigenen Zellen sozusagen eine natürliche Beschichtung des Stents 1, welche das Risiko einer Thrombenbildung gegenüber einem freiliegenden Stent deutlich reduziert. Eine antithrombogene Medikamentierung des Patienten, beispielsweise durch Freisetzung einer antithrombogenen Substanz aus entsprechenden Depots am Stent oder durch systemische Medikation, kann somit entfallen.

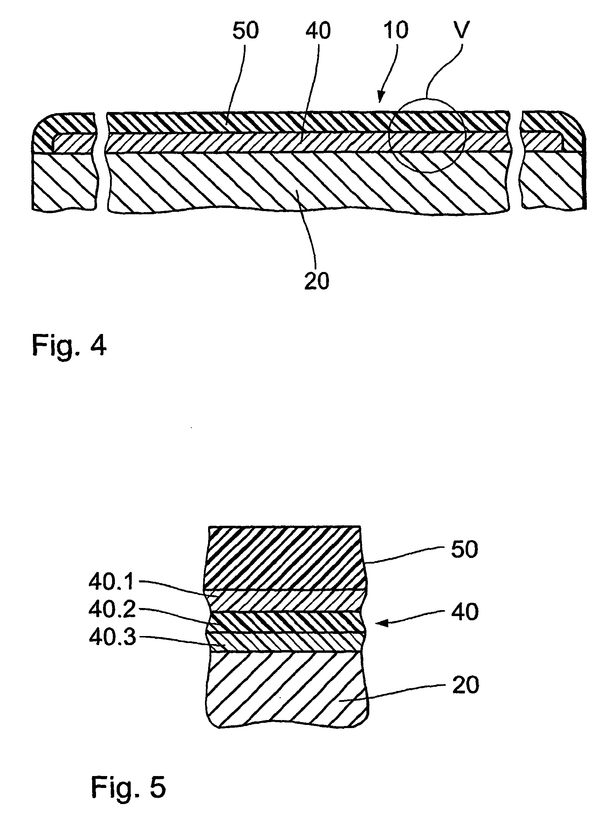

Figur 3 zeigt einen schematischen Teilschnitt durch eine weitere bevorzugte Ausführung eines erfindungsgemäßen Stents 1', welche im wesentlichen der Ausführung aus Figur 1 entspricht, sodass hier lediglich auf die Unterschiede eingegangen werden soll.

Der Unterschied besteht darin, dass die in den ersten Ausnehmungen 3' im Grundkörper 2' versenkten ersten Depots 4' nicht jeweils durch einen gesonderten Deckel abgeschlossen sind, sondern mehrere erste Depots 4' durch eine gemeinsame Abdeckung in Form einer Schicht 5' auf der Oberfläche des Grundkörpers 2' gegenüber der Umgebung des Grundkörpers 2' abgeschirmt sind.

Die Schicht 5' besteht dabei wiederum aus demselben zweiten Stoff, wie er schon für die Deckel der Variante aus Figur 1 Verwendung fand. Ebenso bestehen die ersten Depots 4' aus demselben ersten Stoff, wie er oben zu Figur 1 beschrieben wurde.

Figur 4 zeigt einen schematischen Schnitt durch eine weitere bevorzugte Variante eines erfindungsgemäßen Implantats 10. Dieses weist einen Grundkörper 20 auf, auf dessen Oberfläche ein erstes Depot in Form einer ersten Schicht 40 aus einem ersten Stoff angeordnet ist. Diese erste Schicht 40 ist durch eine Abschirmung in Form einer darüber liegenden zweiten Schicht 50 gegenüber der Umgebung des Grundkörpers 20 abgeschirmt.

Die zweite Schicht 50 besteht wiederum aus einem bioresorbierbaren Polymer, welches sich so langsam zersetzt, dass der zweite Zustand, in dem der Kontakt zwischen der ersten Schicht 40 und der Umgebung des Grundkörpers 20 durch Zersetzung der zweiten Schicht 50 möglich ist, erst nach Ablauf eines ersten Zeitintervalls von drei Wochen eintritt.

Im gezeigten Beispiel besteht der erste Stoff der ersten Schicht 40 wiederum aus einer Matrix aus einem bioresorbierbaren Polymer, an dem Moleküle einer zellproliferationshemmenden ersten Substanz angebunden sind. Die Polymermatrix zersetzt sich unter Einwirkung körpereigener Stoffe, wobei dann auch die Bindung der Moleküle der ersten Substanz zum Matrixpolymer aufgebrochen wird und deren zellproliferationshemmende Wirkung einsetzt. Die Zersetzung erfolgt entsprechend langsam sodass über einen ausreichend langen Zeitraum die zellproliferationshemmende Wirkung durch Freisetzung der ersten Substanz aufrechterhalten wird.

Es versteht sich, dass die erste Schicht bei anderen Varianten der Erfindung auch aus einem ersten Stoff bestehen kann, welcher für sich eine zellproliferationshemmende Wirkung aufweist und damit sozusagen selbst die erste Substanz im Sinne der vorliegenden Erfindung darstellt.

Es versteht sich weiterhin, dass bei anderen Varianten auch vorgesehen sein kann, dass es sich bei den ersten Stoff für die erste Schicht auch um einen biostabilen Werkstoff handeln kann, der seine zellproliferationshemmende Wirkung allein durch den Kontakt mit der Umgebung des Grundkörpers entfaltet.

Bei dem Implantat aus Figur 4 handelt es sich um eine Elektrode, die in der Wandung eines Gefäßes oder einer Wandung des Herzens verankert werden kann. Die erste und zweite Schicht 40 und 50 sind dabei zumindest in demjenigen Bereich der Elektrode 10 angeordnet, der unmittelbaren Kontakt zum Körpergewebe hat, sodass sich dort die schützende Körpergewebelage über der Elektrodenoberfläche ausbilden kann.

In Figur 4 ist lediglich ein Abschnitt der Oberfläche des Grundkörpers 20 mit einer ersten Schicht 40 und einer diese abschirmenden zweiten Schicht 50 versehen.

Es versteht sich, dass bei anderen Varianten mehrere Oberflächenabschnitte aber auch die gesamte Oberfläche mit entsprechenden ersten und zweiten Schichten versehen sein können.

Figur 5 zeigt das Detail V aus Figur 4. Wie Figur 5 zu entnehmen ist, besteht die erste Schicht 40 aus drei unterschiedlichen Teilschichten 40.1, 40.2 und 40.3, welche jeweils aus dem ersten Stoff bestehen. Der Unterschied liegt darin, dass die erste Substanz in dem jeweiligen Teilschichten in unterschiedlicher Konzentration vorliegt. So nimmt die Konzentration der ersten Substanz von der ersten Teilschicht 40.1 über die zweite Teilschicht 40.2 zur dritten Teilschicht 40.3 schrittweise ab. Demgemäß ergibt sich auch hier zunächst eine vergleichsweise starke zellproliferationshemmende Wirkung, die dann jeweils beim Übergang auf den nächste Teilschicht schrittweise abnimmt.

Es versteht sich, dass bei anderen Varianten vorgesehen sein kann, dass ein solcher Konzentrationsgradient der ersten Substanz, gegebenenfalls auch stufenlos, in einer einzigen Schicht erzielt wird. Ebenso versteht es sich, dass die Konzentration der ersten Substanz über die erste Schicht auch in beliebiger anderer Weise variieren kann.

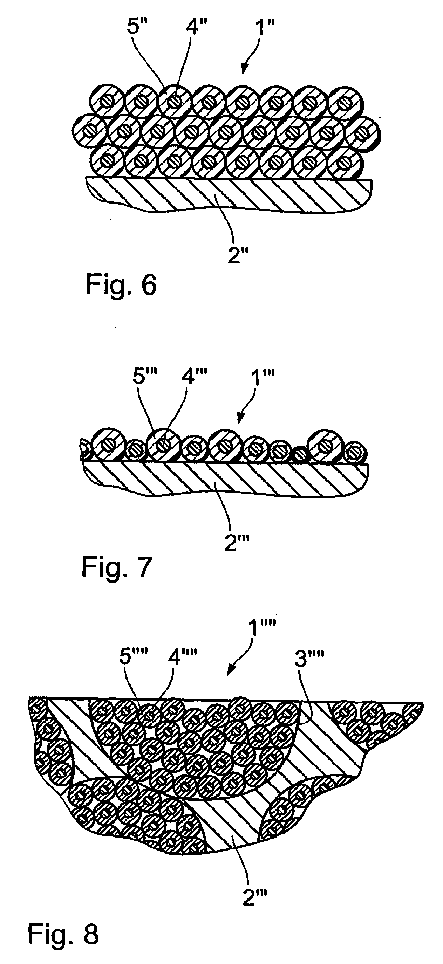

Figur 6 zeigt eine weitere Variante des erfindungsgemäßen Stents 1" mit einem Grundkörper 2", auf dessen Oberfläche in mehreren unregelmäßigen Lagen Mikrokapseln 5" angeordnet sind. Der Grundkörper 2" entspricht dabei bis auf die ersten Ausnehmungen dem Grundkörper aus Figur 1, sodass hierauf nicht näher eingegangen werden soll.

In jeder der Mikrokapseln 5" ist jeweils ein erstes Depot 4" aus einem ersten Stoff eingeschlossen. Die Wandung der Mikrokapseln 5" bildet somit gewissermaßen eine Abschirmung des ersten Stoffes gegenüber der Umgebung des Grundkörpers 2".

Das erste Depot 4" besteht aus einem ersten Stoff, welcher für sich eine zellproliferationshemmende Wirkung aufweist und damit sozusagen selbst die erste Substanz im Sinne der vorliegenden Erfindung darstellt.

Es versteht sich wiederum, dass bei weiteren Varianten der Erfindung der erste Stoff auch, wie vorstehend im Zusammenhang mit anderen Ausführungen beschrieben wurde, aus einer Matrix aus einem bioresorbierbaren Polymer bestehen kann, in der Partikel einer zellproliferationshemmenden ersten Substanz eingelagert sind. Die Polymermatrix zersetzt sich dann wiederum unter Einwirkung körpereigener Stoffe entsprechend langsam, sodass über einen ausreichend langen Zeitraum die zellproliferationshemmende Wirkung die Freisetzung der ersten Substanz aufrechterhalten wird.

Die Wandung der Mikrokapseln 5" besteht wiederum aus einem bioresorbierbaren Polymer, welches sich so langsam zersetzt, dass der zweite Zustand, in dem der Kontakt zwischen den ersten Depot 4" und der Umgebung des Grundkörpers 2" durch Zersetzung der betreffenden Mikrokapsel 5" möglich ist, erst nach Ablauf eines ersten Zeitintervalls von vier Wochen eintritt.

Die Mikrokapseln 5" sind mit der Oberfläche des Grundkörpers 2" sowie untereinander durch einen entsprechenden Klebstoff verbunden. Der Klebstoff und der zweite Stoff weisen eine ausreichende Zähigkeit auf, um Verformungen des Grundkörpers folgen zu können und so ein ungewolltes Lösen der Mikrokapseln 5" vom Stent 1" zu verhindern.

Die Anbringung der Mikrokapseln 5" in mehreren Lagen stellt sicher, dass die erste Substanz über einen längeren Zeitraum hinweg freigesetzt wird. Dies rührt daher, dass aufgrund einer entsprechend dichten Packung der Mikrokapseln 5" zunächst nur die obere Lage der Mikrokapseln 5" durch Einwirkung körpereigener Stoffe zersetzt wird, während die unteren Lagen zunächst weitgehend unbeeinträchtigt bleiben. Die Zersetzung schreitet dann nur relativ langsam bis zur untersten Lage fort.

Figur 7 zeigt eine weitere bevorzugte Variante eines erfindungsgemäßen Stents 1''', der sich in seinem grundsätzlichen Aufbau nicht von demjenigen aus Figur 6 unterscheidet, sodass hier lediglich auf die Unterschiede eingegangen werden soll.

Der Unterschied liegt darin, dass die Mikrokapseln 5''' mit den ersten Depots 4''' in nur einer Lage auf dem Grundkörper 2''' angeordnet sind, dabei aber unterschiedliche Wandstärken aufweisen. Hierdurch ist sichergestellt, dass die erste Substanz nach Ablauf des ersten Zeitintervalls über einen längeren Zeitraum abgegeben wird.

Die Mikrokapseln 5''' mit der geringsten Wandstärke definieren dabei das erste Zeitintervall. Die Freisetzungsraten zu bestimmten Zeiten lassen sich in einfacher Weise z. B. über den jeweiligen Anteil der Mikrokapseln 5''' mit einer bestimmten Wandstärke an der Gesamtmenge der Mikrokapseln 5''' steuern.

Es versteht sich, dass ein vergleichbarer Effekt bei anderen Varianten der Erfindung dadurch erzielt werden kann, dass bei gleicher Wandstärke aller Mikrokapseln unterschiedliche zweite Stoffe verwendet werden, welche sich verschieden schnell zersetzen.

Figur 8 zeigt eine andere bevorzugte Variante eines erfindungsgemäßen Stents 1"", der sich in seinem grundsätzlichen Aufbau nicht von demjenigen aus Figur 6 unterscheidet, sodass auch hier lediglich auf die Unterschiede eingegangen werden soll.

Der Unterschied besteht darin, dass der Grundkörper 2"" eine offenporige Oberfläche aufweist und die Mikrokapseln 5"" mit den ersten Depots 4"" in den durch die Poren 3"" gebildeten ersten Ausnehmungen eingelagert sind. Hierdurch sind die Mikrokapseln 5"" vor der Einwirkung äußerer Kräfte bei Heranführen an den Implantationsort und damit vor einer daraus resultierenden frühzeitigen, mechanischen Abtragung geschützt.

Die in den Figuren 6 bis 8 dargestellten Varianten mit Mikrokapseln weisen ein besonders geringes Risiko einer frühzeitigen Freisetzung der zellproliferationshemmenden ersten Substanz infolge einer Beschädigung der Abschirmung durch den zweiten Stoff auf. Dies rührt daher, dass die einzelnen Abschirmungen, also die Wandungen der Mikrokapseln, auch bei vergleichsweise starken Verformungen des Grundkörpers infolge ihrer Zähigkeit und gegebenenfalls der Zähigkeit der Verbindung solchen Verformungen gut folgen können und demgemäß auch nur relativ geringen Belastungen ausgesetzt sind.

Das Beschädigungsrisiko der Abschirmung kann unter anderem noch dadurch weiter reduziert werden, dass die Verbindung, beispielsweise der dafür verwendete Klebstoff, eine etwas geringere Festigkeit als die Wandung der Mikrokapseln aufweist. Ein Versagen tritt bei zu starken Verformungen dann nur an den Verbindungsstellen ein, sodass die Mikrokapseln selbst intakt bleiben.

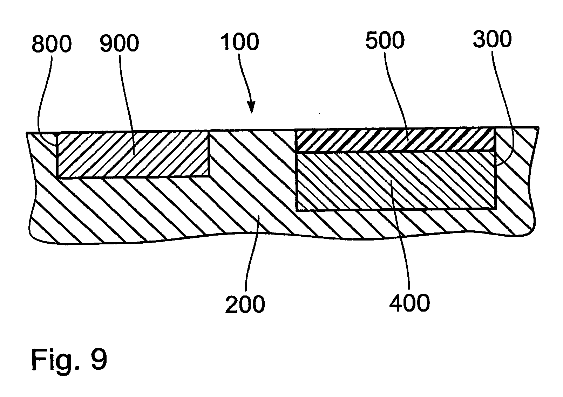

Figur 9 zeigt schließlich eine weitere vorteilhafte Ausgestaltung eines erfindungsgemäßen Implantats 100, welches einen Grundkörper 200 aufweist. In dem Grundkörper 200 ist eine erste Ausnehmung 300 angeordnet, in der ein erstes Depot 400 eingelassen ist. Das erste Depot 400 ist durch einen Deckel 500 gegen die Umgebung des Grundkörpers 200 abgeschirmt.

Das erste Depot 400 besteht aus einem bioresorbierbaren ersten Stoff, der eine zellproliferationshemmende erste Substanz enthält. Der Deckel 500 besteht aus einem bioresorbierbaren zweiten Stoff. Das erste Depot 400 und der Deckel 500 entsprechen in ihrem Aufbau und ihrer Funktion dem ersten Depot 4 bzw. dem Deckel 5 aus Figur 1, sodass hierauf nicht näher eingegangen werden soll, sondern auf die obige Beschreibung zu Figur 1 verwiesen wird.

In dem Grundkörper 200 ist weiterhin eine zweite Ausnehmung 800 vorgesehen, in der ein zweites Depot 900 angeordnet ist. Dieses zweite Depot besteht aus einem dritten Stoff, der eine zellproliferationsfördernde zweite Substanz enthält, die in einer Matrix aus einem bioresorbierbaren Polymer eingelagert ist.

Über den Grundkörper 200 sind gleichmäßig eine Reihe erster Depots 400 und zweiter Depots 900 verteilt, sodass die entsprechenden Wirkungen gleichmäßig das über Implantat 100 verteilt auftreten.

Die Freisetzung der zweiten Substanz erfolgt auf Grund der umgehend einsetzenden Zersetzung der Polymermatrix des dritten Stoffes unmittelbar nach Implantation des Implantats 100. Hierdurch wird die Bildung einer freiliegende Oberflächenabschnitte des Implantats einhüllenden Körpergewebelage, wie sie oben im Zusammenhang mit den Figuren 2A und 2B beschrieben wurde, beschleunigt.

Das zweite Depot 900, insbesondere die Polymermatrix des dritten Stoffes, ist so ausgebildet, dass Zersetzung und damit die Freisetzung der zellproliferationsfördernden zweiten Substanz vor Ablauf des ersten Zeitintervalls, also vor Beginn der Freisetzung der zellproliferationshemmenden ersten Substanz aus dem ersten Depot 400 endet.

Das erste Zeitintervall ist durch entsprechende Gestaltung des Deckels 500, insbesondere durch entsprechende Auswahl des dafür verwendeten zweiten Stoffes, infolge der beschleunigten Bildung der einhüllenden Körpergewebelage kürzer als bei den vorbeschriebenen Varianten. Es beträgt etwa eine Woche.

Bei alternativen Ausführungsformen kann ein vergleichbarer Effekt dadurch erzielt werden, dass in einer Konfiguration, wie sie zu einer der vorstehend beschriebenen Figuren dargestellt ist, der zweite Stoff eine entsprechend zellproliferationsfördernde zweite Substanz umfasst.

Obwohl das erfindungsgemäße Implantat vorstehend fast ausschließlich anhand von Stents beschrieben wurde, versteht es sich, dass sich die Erfindung auch auf beliebige andere intravaskuläre oder intrakardiale Implantatstypen, wie beispielsweise Elektroden etc., anwenden lässt.

## Patentansprüche

1. Implantat zum Einsetzen in einen Abschnitt des menschlichen oder tierischen Blutkreislaufs, insbesondere Stent, mit einem Grundkörper (2; 2'; 2"; 2"'; 2""; 20; 200), wenigstens einem mit dem Grundkörper (2; 2'; 2"; 2'"; 2""; 20; 200) verbundenen, wenigstens eine zellproliferationshemmende erste Substanz umfassenden ersten Stoff und einem sich im implantierten Zustand zersetzenden zweiten Stoff, welcher derart angeordnet und ausgebildet ist, dass er in einem ersten Zustand vor Beginn seiner Zersetzung den Kontakt zwischen der ersten Substanz und der Umgebung des Grundkörpers (2; 2'; 2"; 2"' ; 2""; 20; 200) verhindert und in einem zweiten Zustand nach Beginn seiner Zersetzung den Kontakt zwischen der ersten Substanz und der Umgebung des Grundkörpers (2; 2'; 2"; 2'"; 2""; 20; 200) ermöglicht, **dadurch gekennzeichnet, dass** der zweite Stoff weiterhin derart ausgebildet ist, dass der zweite Zustand erst nach einem ersten Zeitintervall erreicht wird, welches zur Bildung einer wenigstens einen Abschnitt der Oberfläche des Grundkörpers (2; 2'; 2"; 2"'; 2""; 20; 200) einhüllenden Körpergewebelage (7) ausreicht.

2. Implantat nach Anspruch 1, **dadurch gekennzeichnet, dass** das erste Zeitintervall zur Bildung einer einen Abschnitt der Oberfläche des Grundkörpers (2; 2'; 2"; 2"'; 2""; 20; 200) bedeckenden Hülle (7) aus Körpergewebe, insbesondere aus glatten Muskelzellen, ausreicht.

3. Implantat nach Anspruch 1 oder 2, **dadurch gekennzeichnet, dass** das erste Zeitintervall wenigstens eine Woche, vorzugsweise zwei bis vier Wochen beträgt.

4. Implantat nach einem der vorhergehenden Ansprüche, **dadurch gekennzeichnet, dass** wenigstens ein erstes Depot (4; 4'; 4"; 4'"; 4""; 40; 400) des ersten Stoffes vorgesehen ist, welches im ersten Zustand durch eine Abdeckung (5; 5'; 5"; 5"'; 5""; 50; 500) des zweiten Stoffes gegenüber der Umgebung des Grundkörpers (2; 2'; 2"; 2"'; 2""; 20; 200) abgeschirmt ist.

5. Implantat nach Anspruch 4, **dadurch gekennzeichnet, dass** das erste Depot von einer mit dem Grundkörper (20) verbundenen ersten Schicht (40) gebildet ist und die Abdeckung von einer die erste Schicht (40) bedeckenden zweiten Schicht (50) gebildet ist.

6. Implantat nach Anspruch 4, **dadurch gekennzeichnet, dass** das erste Depot (4; 4'; 4""; 400) in einer ersten Ausnehmung (3; 3'; 3""; 300) des Grundkörpers (2; 2'; 2""; 200) angeordnet ist, wobei die Abdeckung von einem die erste Ausnehmung (3; 3'; 3""; 300) verschließenden Deckel (5; 5'; 5""; 500) gebildet ist.

7. Implantat nach Anspruch 6, **dadurch gekennzeichnet, dass** der Grundkörper (2"") zur Ausbildung einer Anzahl erster Ausnehmungen (3"") zumindest abschnittsweise eine poröse Oberfläche aufweist.

8. Implantat nach Anspruch 4, **dadurch gekennzeichnet, dass** das erste Depot (4"; 4"'; 4"") in einer die Abdeckung bildenden Mikrokapsel (5"; 5"'; 5"") eingeschlossen ist, welche mit dem Grundkörper (2"; 2"'; 2"") verbunden ist.

9. Implantat nach einem der vorhergehenden Ansprüche, **dadurch gekennzeichnet, dass** zur Förderung der Zellproliferation vor Ablauf des ersten Zeitintervalls ein mit dem Grundkörper (200) verbundener dritter Stoff vorgesehen ist, der eine zellproliferationsfördernde zweite Substanz umfasst.

10. Implantat nach Anspruch 9, **dadurch gekennzeichnet, dass** der dritte Stoff derart ausgebildet ist, insbesondere die Konzentration der zweiten Substanz derart gewählt ist, dass die zellproliferationsfördernde Wirkung vor oder mit Ablauf des ersten Zeitintervalls im wesentlichen abgeschlossen ist.

11. Implantat nach einem der vorhergehenden Ansprüche, **dadurch gekennzeichnet, dass** es als Stent (1; 1'; 1"; 1"'; 1 ""), insbesondere Koronarstent, ausgebildet ist.

## Claims

1. An implant for insertion in a section of the human or animal blood circulation, in particular a stent, with a base body (2; 2'; 2"; 2"', 2""; 20; 200), at least one first material connected to the base body (2; 2'; 2"; 2"'; 2""; 20; 200) comprising at least one cell proliferation inhibiting first substance, and a second material that decomposes in the implanted condition, which material is arranged and designed so that in a first condition it prevents contact between the first substance and the area surrounding the base body (2; 2'; 2"; 2"', 2"", 20; 200) before its decomposition commences, and allows contact between the first substance and the area surrounding the base body (2; 2'; 2"; 2"', 2""; 20; 200) after commencement of its decomposition, **characterised in that** the second material is also designed so that the second condition is not reached until after a first time interval, which is sufficient for the formation of a body tissue layer (7) enclosing at least one section of the surface of the base body (2; 2'; 2"; 2"', 2""; 20; 200).

2. The implant according to Claim 1, **characterised in that** the first time interval is sufficient for the formation of an envelope (7) of body tissue, in particular of smooth muscle cells, covering one section of the surface of the base body (2; 2'; 2"; 2"', 2""; 20; 200).

3. The implant according to Claim 1 or 2, **characterised in that** the first time interval is at least one week, preferably two to four weeks.

4. The implant according to one of the preceding claims, **characterised in that** at last one first deposit (4; 4'; 4"; 4"'; 4""; 40; 400) of the first material is provided, which deposit, in the first condition, is protected by a cover (5; 5'; 5"; 5"'; 5""; 50; 500) of the second material against the area surrounding the base body (2; 2'; 2"; 2"', 2""; 20; 200).

5. The implant according to Claim 4, **characterised in that** the first deposit is formed from a first layer (40) connected to the base body (20), and **in that** the cover is formed from a second layer (50) covering the first layer (40).

6. The implant according to Claim 4, **characterised in that** the first deposit (4; 4'; 4""; 400) is arranged in a first recess (3; 3'; 3""; 300) of the base body (2; 2'; 2""; 200), wherein the cover is formed from a lid (5; 5'; 5""; 500) sealing the first recess (3; 3', 3""; 300).

7. The implant according to Claim 6, **characterised in that** the base body (2"") has at least in sections a porous surface for forming a number of first recesses (3"").

8. The implant according to Claim 4, **characterised in that** the first deposit (4"; 4"'; 4"") is enclosed in a microcapsule (5"; 5"'; 5"") which is connected to the base body (2"; 2"'; 2"").

9. The implant according to one of the preceding claims, **characterised in that** a third material connected to the base body (200) is provided for promoting the cell proliferation before the end of the first time interval, which material comprises a cell proliferation promoting second substance.

10. The implant according to Claim 9, **characterised in that** the third material is designed, and in particular the concentration of the second substance is selected, so that the cell proliferation promoting action is essentially terminated before or at the end of the first time interval.

11. The implant according to one of the preceding claims, **characterised in that** it is designed as a stent (1; 1'; 1"; 1"'; 1""), in particular a coronary stent.

## Revendications

1. Implant pour l'insertion dans une partie de la circulation sanguine humaine ou animale, en particulier un stent, avec un corps de base (2 ; 2' ; 2" ; 2"'; 2"" ; 20 ; 200) au moins un premier produit relié au corps de base (2 ; 2' ; 2" ; 2"' ; 2"" ; 20 ; 200) et comprenant au moins une première substance inhibant la prolifération cellulaire et un second produit se décomposant dans l'état implanté, qui est disposé et réalisé de telle sorte que, dans le premier état antérieur au début de sa décomposition, il empêche le contact entre la première substance et l'environnement du corps de base (2 ; 2' ; 2" ; 2"' ; 2"" ; 20 ; 200) et, dans le second état postérieur au début de sa décomposition, il permet le contact entre la première substance et l'environnement du corps de base (2 ; 2' ; 2" ; 2"' ; 2"" ; 20 ; 200) **caractérisé en ce que** le second produit est conçu également de telle sorte que le second état n'est obtenu qu'après un premier intervalle de temps, qui est suffisant pour la formation d'une couche de tissu du corps (7) enveloppant au moins une partie de la surface du corps de base (2 ; 2' ; 2" ; 2"'; 2"" ; 20 ; 200).

2. Implant selon la revendication 1, **caractérisé en ce que** le premier intervalle de temps suffit pour la formation d'une enveloppe (7) recouvrant une partie de la surface du corps de base (2 ; 2' ; 2" ; 2"' ; 2"" ; 20 ; 200) à base du tissu du corps, en particulier à base de cellules musculaires lisses.

3. Implant selon la revendication 1 ou 2, **caractérisé en ce que** le premier intervalle de temps est d'au moins une semaine, de préférence de deux à quatre semaines.

4. Implant selon l'une quelconque des revendications précédentes, **caractérisé en ce qu'**au moins un premier dépôt (4 ; 4' ;4" ; 4"' ; 4"" ; 40 ; 400) du premier produit est prévu, lequel est protégé dans le premier état par un revêtement (5 ; 5' ; 5" ; 5"' ; 5"" ; 50 ; 500) du second produit par rapport à l'environnement du corps de base (2 ; 2' ; 2" ; 2"' ; 2"" ; 20 ; 200).

5. Implant selon la revendication 4, **caractérisé en ce que** le premier dépôt est formé par une première couche (40) reliée au corps de base (20) et le revêtement est formé par une seconde couche (50) recouvrant la première couche (40).

6. Implant selon la revendication 4, **caractérisé en ce que** le premier dépôt (4 ; 4' ;4" ; 4"'; 4"" ; 40 ; 400) est disposé dans un premier évidement (3 ; 3' ; 3"" ; 300) du corps de base (2 ; 2' ; 2"" ; 200), le revêtement étant formé par un couvercle (5 ; 5' ; 5"" ; 500) fermant le premier évidement (3 ; 3' ; 3"" ; 300).

7. Implant selon la revendication 6, **caractérisé en ce que** le corps de base (2"") présente au moins par endroits une surface poreuse pour la formation d'un certain nombre de premiers évidements (3"").

8. Implant selon la revendication 4, **caractérisé en ce que** le premier dépôt (4", 4"', 4"") est inclus dans une microcapsule (5", 5"', 5"") formant le revêtement, qui est reliée au corps de base (2", 2"', 2"").

9. Implant selon l'une quelconque des revendications précédentes, **caractérisé en ce que**, pour favoriser la prolifération cellulaire avant l'expiration du premier intervalle de temps, il est prévu un troisième produit relié au corps de base (200) qui comprend une seconde substance favorisant la prolifération cellulaire.

10. Implant selon la revendication 9, **caractérisé en ce que** le troisième produit est conçu et en particulier la concentration de la seconde substance est choisie de telle sorte que l'effet favorisant la prolifération cellulaire est sensiblement terminé avant ou avec l'expiration du premier intervalle de temps.

11. Implant selon l'une quelconque des revendications précédentes, **caractérisé en ce qu'**il est conçu comme stent (1 ; 1' ; 1" ; 1"' ; 1""), en particulier un stent coronarien.
